# EUROPEAN PATENT APPLICATION

(11) **EP 2 269 597 A1**
(43) Date of publication of application: **05.01.2011**
(21) Application number: 09008143.1
(22) Date of filing: 22.06.2009
(51) Int. Cl.: A61K 31/00, A61K 39/395, C07K 16/28, G01N 33/53

(54) **CLEC-2 is an essential platelet activating receptor in hemostasis and thrombosis**

(71) Applicant: CSL Behring GmbH, 35041 Marburg (DE); Julius-Maximilians-Universität Würzburg, 97070 Würzburg (DE)
(72) Inventor: Nieswandt, Bernhard, 97246 Eibelstadt (DE); May, Frauke, 97072 Würzburg (DE)

(57) **Abstract**

The present invention relates to a pharmaceutical composition comprising an inhibitor of C-type lectin-like receptor 2 (CLEC-2) or an inhibitor of an activator of CLEC-2 and optionally a pharmaceutically acceptable carrier, excipient and/or diluent. Furthermore, the present invention relates to an inhibitor of C-type lectin-like receptor 2 (CLEC-2) or an inhibitor of an activator of CLEC-2 for use in treating or preventing a disorder related to venous or arterial thrombus formation or as a lead compound for developing a drug for treating or preventing a disorder related to venous or arterial thrombus formation. The present invention also relates to a method of treating and/or preventing a disorder related to venous or arterial thrombus formation comprising administering a pharmaceutically effective amount of an inhibitor of C-type lectin-like receptor 2 (CLEC-2) or an inhibitor of an activator of CLEC-2 to a subject in need thereof. The present invention further relates to methods of identifying a compound suitable as a lead compound and/or as a medicament for the treatment and/or prevention of a disorder related to venous or arterial thrombus formation.

## Description

The present invention relates to a pharmaceutical composition comprising an inhibitor of C-type lectin-like receptor 2 (CLEC-2) or an inhibitor of an activator of CLEC-2 and optionally a pharmaceutically acceptable carrier, excipient and/or diluent. Furthermore, the present invention relates to an inhibitor of C-type lectin-like receptor 2 (CLEC-2) or an inhibitor of an activator of CLEC-2 for use in treating or preventing a disorder related to venous or arterial thrombus formation or as a lead compound for developing a drug for treating or preventing a disorder related to venous or arterial thrombus formation. The present invention also relates to a method of treating and/or preventing a disorder related to venous or arterial thrombus formation comprising administering a pharmaceutically effective amount of an inhibitor of C-type lectin-like receptor 2 (CLEC-2) or an inhibitor of an activator of CLEC-2 to a subject in need thereof. The present invention further relates to methods of identifying a compound suitable as a lead compound and/or as a medicament for the treatment and/or prevention of a disorder related to venous or arterial thrombus formation.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Platelet plug formation is a multi-step process that involves the concerted action of multiple membrane receptors and intracellular signaling pathways. Under conditions of elevated shear as found in arterioles or stenosed arteries, the rapid on-set of interaction between glycoprotein (GP) Ib-V-IX and von Willebrand factor (vWF) immobilized on collagen is crucial for the initial tethering of flowing platelets (5). This interaction is, however, not stable and must be followed by the stimulation of platelet receptors that mediate cellular activation resulting in calcium mobilization, shape change, upregulation of integrin affinity, release of secondary agonists and coagulant activity of the cells (2;6;7). Two major classes of activatory receptors exist in platelets. Soluble agonists such as thrombin, adenosine diphosphate (ADP) and thromboxane A2 (TxA₂) stimulate receptors that couple to heterotrimeric G proteins (G_{q}, G_{12/13}, G_{i/z}) and activate downstream effectors (8). The other pathway is similar to that used by immunoreceptors and involves sequential activation of Src and Syk family tyrosine kinases which orchestrate a downstream signaling cascade that is regulated through the interaction of several adaptor proteins, including LAT and SLP-76 and leads to activation of effector enzymes, including phosphatidylinositol-3-kinases and phospholipase (PL) Cγ2. This pathway is triggered by the major activatory platelet collagen receptor, GPVI, which signals via the *immunoreceptor tyrosine-based activation motif* (ITAM)-bearing Fc receptor γ-chain (9;10) or by CLEC-2, where signaling is initiated by tyrosine phosphorylation of a single YXXL motif in its cytoplasmic tail (11;12). While the mechanisms underlying platelet activation on the ECM are well explored, it is less clear which receptors mediate cellular activation in a growing thrombus.

Platelet aggregation is a key mechanism for normal hemostasis limiting blood loss after tissue trauma (1), but at sites of atherosclerotic plaque rupture it may also lead to arterial occlusion and embolism causing myocardial infarction or stroke (2;3). Therefore, the inhibition of platelet function has become an important strategy for prevention and treatment of ischemic cardio- and cerebrovascular events (4).

Despite the fact that thrombus formation leads to some of the most frequently occurring diseases in humans und despite extensive basic and clinical research that has been carried out in the field of thrombosis over decades, medicaments that have been registered and are presently available for patients are unsatisfactory for a variety of reasons. One problem common to all anti-coagulants presently used in clinics is their association with an increased risk of serious bleeding. These include heparins, Cumarins, direct thrombin inhibitors such as hirudin, as well as aspirin, P2Yi2 inhibitors such as Clopidogrel and GPIIb/IIIa inhibitors such as abciximab (ReoPro). In addition, for some of these known inhibitors such as aspirin or the P2Yi2 inhibitor Clopidogrel, many patients have been described as low or nonresponders (44).

The technical problem underlying the present invention is thus the provision of alternative and/or improved means and methods for treating diseases based on thrombus formation.

The solution to this technical problem is achieved by providing the embodiments characterised in the claims.

Accordingly, the present invention relates to a pharmaceutical composition comprising an inhibitor of C-type lectin-like receptor 2 (CLEC-2) or an inhibitor of an activator of CLEC-2 and optionally a pharmaceutically acceptable carrier, excipient and/or diluent.

The term "pharmaceutical composition" in accordance with the present invention relates to a composition for administration to a patient, preferably a human patient. The pharmaceutical composition of the invention comprises at least one, such as at least two, e.g. at least three, in further embodiments at least four such as at last five of the above mentioned inhibitors. The invention also envisages mixtures of inhibitors of CLEC-2 and inhibitors of an activator of CLEC-2. In cases where more than one inhibitor is comprised in the composition it is understood that none of these inhibitors has an inhibitory effect on the other inhibitors also comprised in the composition.

The composition may be in solid, liquid or gaseous form and may be, inter alia, in a form of (a) powder(s), (a) tablet(s), (a) solution(s) or (an) aerosol(s).

It is preferred that said pharmaceutical composition comprises a pharmaceutically acceptable carrier, excipient and/or diluent, Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the bloodstream such as a brain or coronary artery or directly into the respective tissue The compositions of the invention may also be administered directly to the target site, e.g., by biolistic delivery to an external or internal target site, like the brain. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize thrombus formation or to reduce thrombus size, such as for example anti-coagulants as described in WO2006/066878, which is specifically incorporated herein in its entirety. Since the pharmaceutical preparation of the present invention relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

The term "CLEC-2" relates to the "C-type lectin-like receptor 2" and both terms are used interchangeably herein. CLEC-2 (GenBank database accession no. AF124841; GeneID: 51266) is a C-type lectin-like type II transmembrane receptor that was originally identified in immune cells (13;14) and only recently revealed to be expressed in platelets. There are two known transcript variants of human CLEC-2, the mRNA sequences of which can be found e.g. under the NCBI accession numbers NM_001099431.1 for transcript variant 1 and NM_016509.3 for transcript variant 2. CLEC-2 has been suggested to be involved in hematogenous tumor metastasis as the receptor mediates tumor cell-induced platelet activation, a process known to significantly promote tumor cell spreading (19;20) by interacting with the type I transmembrane sialomucin-like glycoprotein podoplanin (aggrus) on the tumor cell surface(21). In addition, CLEC-2 has been identified as an attachment factor for human immunodeficiency virus type 1 (HIV-1) that mediates the capture and transfer of infectious HIV-1 by platelets, making it a potentially important determinant of virus spread in infected humans (22). In addition, the receptor has been reported to play a role in phagocytic activity of neutrophils (23). However, the role of CLEC-2 for platelet activation during hemostasis and in the course of thrombotic events has not yet been defined (24).

The term "inhibitor" in accordance with the present invention refers to an inhibitor that reduces the biological function of a particular target protein. An inhibitor may perform any one or more of the following effects in order to reduce the biological function of the protein to be inhibited: (i) the transcription of the gene encoding the protein to be inhibited is lowered, i.e. the level of mRNA is lowered, (ii) the translation of the mRNA encoding the protein to be inhibited is lowered, and (iii) the protein performs its biochemical function with lowered efficiency in the presence of the inhibitor.

Compounds falling in class (i) include compounds interfering with the transcriptional machinery and/or its interaction with the promoter of said gene and/or with expression control elements remote from the promoter such as enhancers. Compounds of class (ii) comprise antisense constructs and constructs for performing RNA interference (e.g. siRNA, shRNA, siRNA) well known in the art (see, e.g. Zamore (2001) Nat. Struct. Biol. 8(9), 746; Tuschl (2001) Chembiochem. 2(4), 239). Compounds of class (iii) interfere with the molecular function of the protein to be inhibited, in the present case of the CLEC-2 receptor binding to a ligand as well as its down-stream signalling activity and its activity on platelet aggregation. Accordingly, active site binding compounds, in particular compounds capable of binding to the ligand binding site are envisaged.

The inhibitor, in accordance with the present invention, can in certain embodiments be provided as a proteinaceous compound or as a nucleic acid molecule encoding the inhibitor. For example, the nucleic acid molecule encoding the inhibitor may be incorporated into an expression vector comprising regulatory elements, such as for example specific promoters, and thus can be delivered into a cell. Method for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor into an expression vector allows to permanently elevate the level of the encoded inhibitor in any cell or a subset of selected cells of the recipient. Thus, a tissue- and/or time-dependent expression of the inhibitor can be achieved, for example restricted to blood cells, Thus, in a preferred embodiment, the inhibitor is a thrombocyte-specific inhibitor.

The term "inhibitor of CLEC-2" in accordance with the present invention refers to an inhibitor that reduces the biological function of CLEC-2. Biological function denotes in particular any known biological function of CLEC-2 including functions elucidated in accordance with the present invention. Examples of said biological function are thrombus formation, e.g. formation of stable platelet-platelet contacts under flow conditions or intravascular aggregate formation at sites of inflammation or injury. All these functions can be tested for either using any of a variety of standard methods known in the art, such as for example standard aggregometry, *ex vivo* flow adhesion studies, flow cytometric methods for determination of platelet activation, *in vivo* thrombosis models in animals or on the basis of the teachings of the examples provided below, optionally in conjunction with the teachings of the documents cited therein.

In a preferred embodiment, the inhibitor reduces the biological function of CLEC-2 by at least 50%, preferably by at least 75%, more preferred by at least 90% and even more preferred by at least 95% such as at least 98% or even by 100%. The term reduction by at least, for example 75%, refers to a decreased biological function such that CLEC-2 looses 75% of its function and, consequently, has only 25% activity remaining as compared to CLEC-2 that is not inhibited.

The term "inhibitor of an activator of CLEC-2" refers to inhibitors that do not directly interact with CLEC-2 but with molecules that directly or indirectly activate one or more of the biological functions of CLEC-2 and preferably one or more of those functions referred to above or elsewhere in this specification. Non-limiting examples of molecules that directly or indirectly activate one or more of the biological functions of CLEC-2 include podoplanin, rhodocytin or derivatives thereof. The inhibition values referred to above for inhibitors of CLEC-2 mutatis mutandis apply to inhibitors of an activator of CLEC-2.

The function of any of the inhibitors referred to in the present invention may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibit biological activity, said mixture of test compounds may be de-convoluted to identify the one or more test compounds in said mixture giving rise to the observed biological activity.

The determination of binding of potential inhibitors can be effected in, for example, any binding assay, preferably biophysical binding assay, which may be used to identify binding test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay.

In cases where the inhibitor acts by decreasing the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidiumbromide or SybrGreen. A lower band intensity of the sample treated with the inhibitor as compared to a non-treated sample indicates that the inhibitor successfully inhibits the expression of the protein.

Real-time PCR employs a specific probe, in the art also referred to as TaqMan probe, which has a reporter dye covalently attached at the 5' end and a quencher at the 3' end, After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SybrGreen for monitoring the de novo synthesis of double stranded DNA molecules.

Methods for the determination of the expression of a protein on the amino acid level include but are not limited to western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique.

In accordance with the present invention it was surprisingly found that the recently identified platelet membrane glycoprotein CLEC-2 plays a fundamental role in thrombus formation in hemostasis and thrombosis. These results were surprising as the only known endogenous ligand of the receptor, podoplanin, is mainly expressed in epithelial and tumor cells and can therefore not account for CLEC-2 activation at sites of vascular injury (21;24).

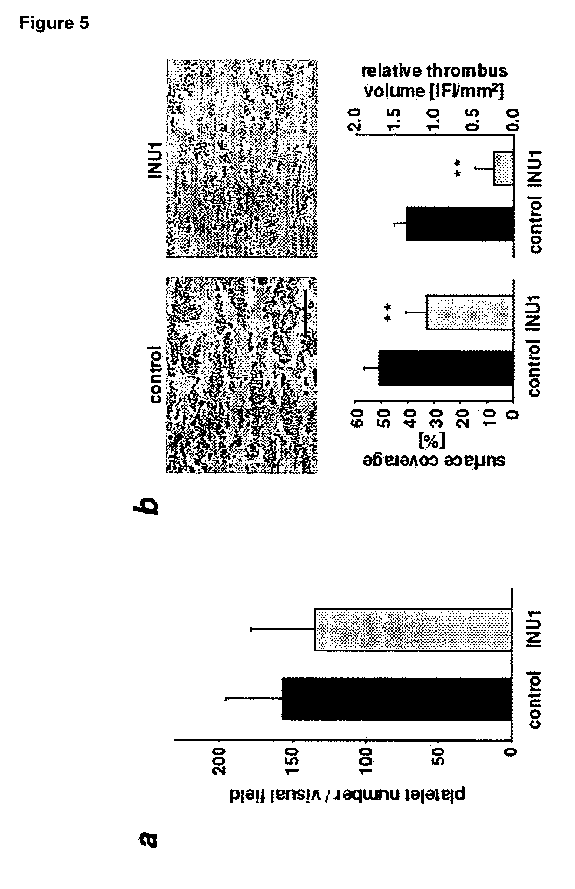

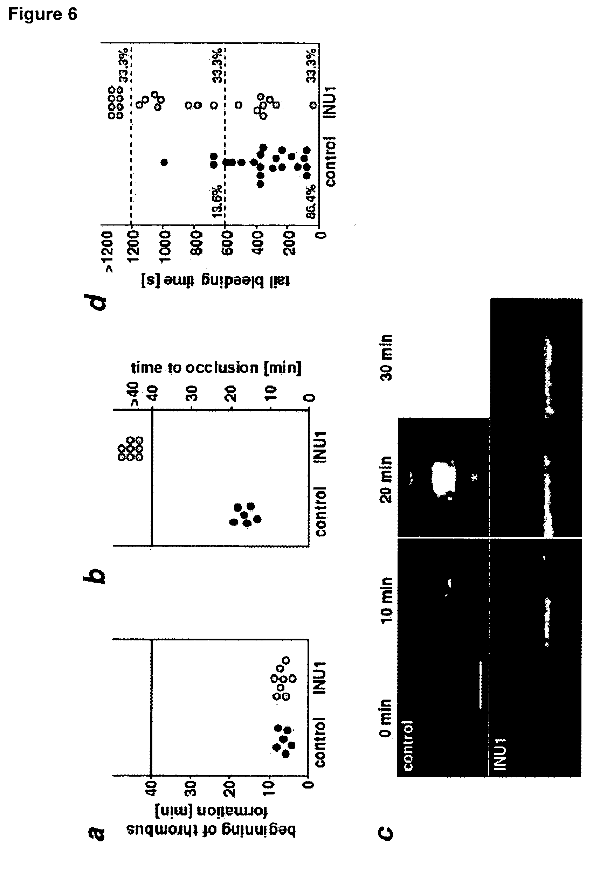

Thrombus formation under flow conditions *in vitro* and also *in vivo* was found herein to be severely defective in the antibody-induced absence of CLEC-2. This strongly suggests that the ligand(s) of the receptor are present in plasma or they may be presented on the surface of or released by (activated) platelets. CLEC-2 ligands could also be present at the injured vessel wall but this may not be crucial for attachment of the first layer of platelets as indicated by unaltered formation of small aggregates at sites of injury in CLEC-2-deficient mice *in vivo* (Fig. 6). This notion is also supported by the observation that CLEC-2-deficient platelets can attach normally to collagen under high shear flow conditions (Fig. 5A), a process known to be driven mainly by GPIb, GPVI and integrins a2β1 and αIIbβ3 (6;7;29). Rather, CLEC-2-dependent signaling appears to be required for establishing (activation-dependent) stable platelet-platelet contacts under flow conditions as revealed by the defective transition of newly recruited platelets to firm adhesion on the surface of collagen-adherent platelets. This process also appears to be crucial for stable thrombus formation *in vivo* as revealed by the constant release of individual platelets and frequent embolization of small aggregates from the surface of the developing thrombus in CLEC-2-deficient mice. As a consequence, no occlusion was seen in these animals, thus identifying CLEC-2 as a novel target for antithrombotic therapy.

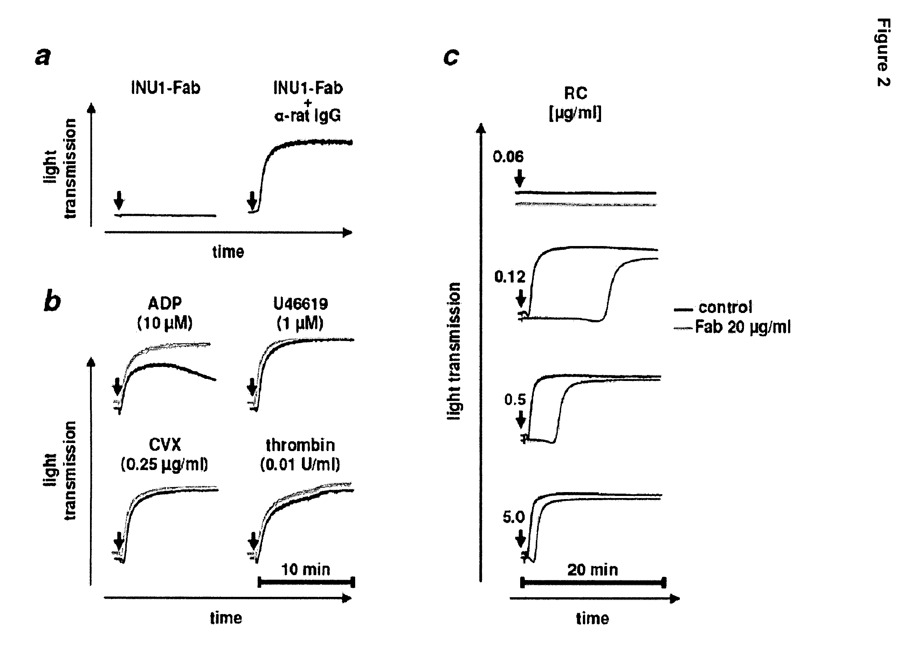

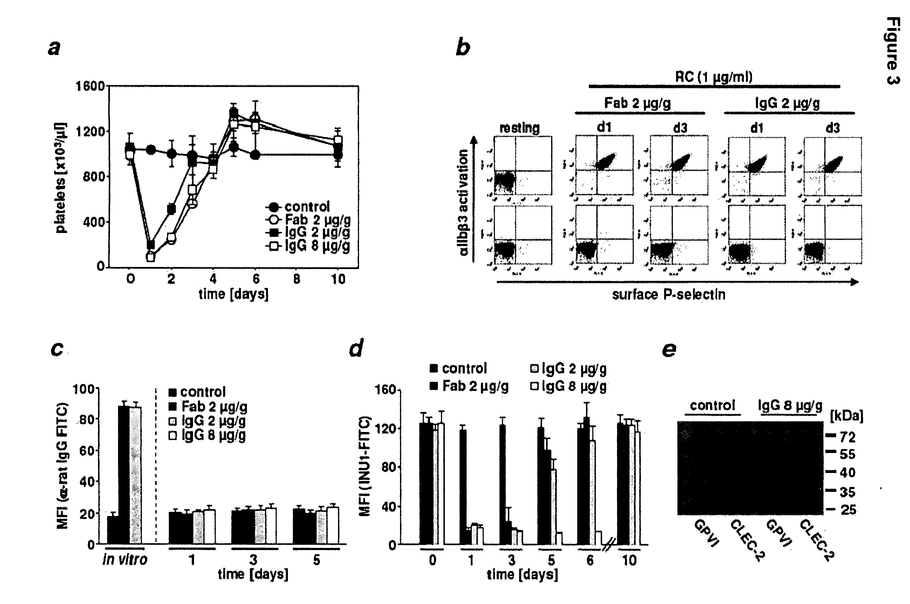

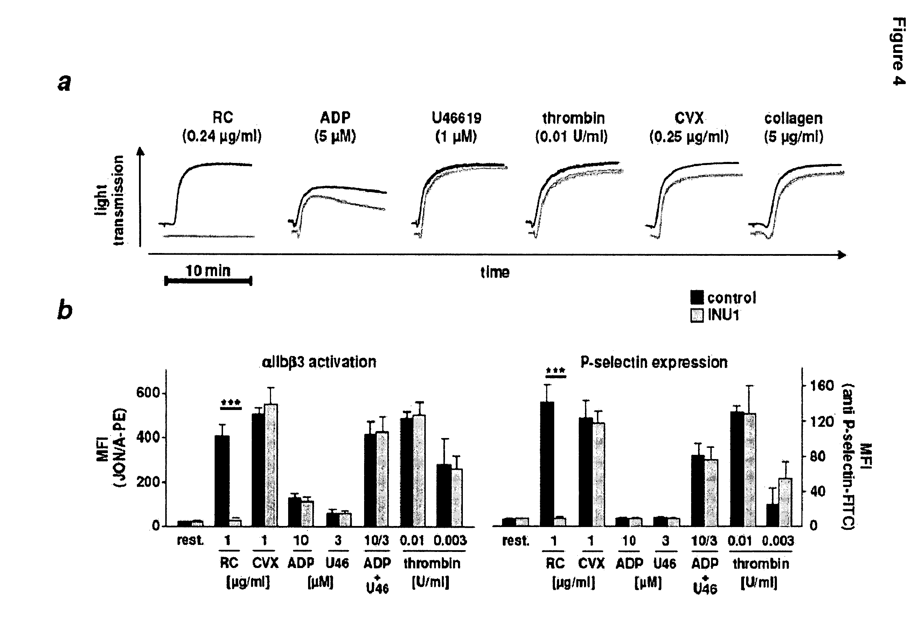

It was further shown in accordance with the present invention that CLEC-2 can indeed be specifically targeted and functionally inactivated by the antibody INU1 *in vivo.* Remarkably, this *in vivo* inhibition by far exceeded the inhibitory potential of the antibody *in vitro* as it was not based on pure blockade of the receptor. Rather, INU1 induced the irreversible loss of CLEC-2 from circulating platelets, a process previously only reported for GPVI in mouse and human platelets (30;31). The complete loss of functional CLEC-2 in platelets of INU1-treated mice was confirmed by different approaches. Firstly, these platelets were completely refractory to activation with rhodocytin (Fig. 3, 4) whereas Fab fragments of the antibody only had a limited inhibitory potential *in vitro* (Fig. 2). Secondly, flow cytometric analyses confirmed the complete loss of CLEC-2 from the surface of circulating platelets within 24 hours after INU1 injection and thirdly, immunoprecipitation confirmed a prolonged absence of the protein for at least 5 days (Fig. 3).

The *in vivo* studies thus show that treatment of mice with an antibody against CLEC-2 induces a specific CLEC-2 deficiency in platelets for a prolonged period of time which is associated with profound protection of the animals from occlusive thrombus formation. Furthermore, whereas lack of CLEC-2 is associated with an increase in bleeding times (Fig. 6), this effect is moderate compared to the bleeding time prolongations induced by integrin αIIbβ3 or GPIbα inhibition (40). Thus, it is speculated on the grounds of these results that anti-CLEC-2 therapy might be associated with a relatively low risk of clinical hemorrhage.

Taken together, it has surprisingly been found that CLEC-2-deficient platelets displayed normal adhesion under flow but subsequent aggregate formation was severely defective in vitro and in vivo. As a consequence, CLEC-2 deficiency was associated with profound protection from occlusive arterial thrombus formation. These results reveal an essential function of CLEC-2 in hemostasis and thrombosis. Thus, CLEC-2 is an essential mediator of platelet activation *in vitro* and *in vivo* and represents an interesting antithrombotic target that can be functionally inactivated in vivo. These findings can serve as a basis for the development of a new generation of powerful, yet safe, agents for prophylaxis and treatment of ischemic cardiovascular events.

The present invention further relates to an inhibitor of C-type lectin-like receptor 2 (CLEC-2) or an inhibitor of an activator of CLEC-2 for use in treating or preventing a disorder related to venous or arterial thrombus formation or as a lead compound for developing a drug for treating or preventing a disorder related to venous or arterial thrombus formation.

Where the inhibitor is used as a lead compound for the development of a drug for treating or preventing a disorder related to venous or arterial thrombus formation, the following developments are considered: modifications to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiff's bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000). Those lead compounds will also allow for the development of novel, highly effective, yet safe antithrombotics.

The term "disorder related to venous thrombus formation" as used herein relates to diseases caused by or related to blood clot formation within a vein, Since the veins return blood to the heart, breaking off of a piece of a blood clot formed in a vein can lead to its being transported to the right side of the heart, and from there into the lungs, leading to pulmonary embolism as described below. Superficial venous thromboses can cause discomfort but generally do not cause serious consequences, unlike the deep vein thromboses (DVTs) that form in the deep veins of the legs or in the pelvic veins.

The term "disorder related to arterial thrombus formation" as used herein relates to diseases caused by or related to the formation of a thrombus within an artery. In most cases, formation of a thrombus within an artery follows rupture of atheroma, and is therefore referred to as atherothrombosis.

The disorder related to venous or arterial thrombus formation is selected from, but not limited to, a group consisting of myocardial infarction, stroke, ischemic stroke, pulmonary thromboembolism, peripheral artery disease (PAD), arterial thrombosis and venous thrombosis.

The present invention further relates to a method of treating and/or preventing a disorder related to venous or arterial thrombus formation comprising administering a pharmaceutically effective amount of an inhibitor of C-type lectin-like receptor 2 (CLEC-2) or an inhibitor of an activator of CLEC-2 to a subject in need thereof.

In a preferred embodiment of the pharmaceutical composition or the inhibitor or the method of the invention, the inhibitor is an antibody or a fragment or derivative thereof, an aptamer, a siRNA, a shRNA, a miRNA, a ribozyme, an antisense nucleic acid molecule or a small molecule.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of CLEC-2 or an epitope of an activator of CLEC-2 (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "protein" as used herein describes a group of molecules consisting of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc. The terms "peptide" and "protein" (wherein "protein" is interchangeably used with "polypeptide") also refer to naturally modified peptides/proteins wherein the modification is effected e.g. by glycosylation, acetylation, phosphorylation and the like. Such modifications are well-known in the art.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of CLEC-2 or an inhibitor of an activator of CLEC-2.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" according to the present invention may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays.

In another preferred embodiment of the pharmaceutical composition or the inhibitor or the method of the invention, the activator of CLEC-2 is selected from the group consisting of podoplanin, rhodocytin or derivatives thereof.

The terms "podoplanin" and "rhodocytin" as used herein refer to ligands of the CLEC-2 receptor. So far, podoplanin is the only known endogenous ligand for CLEC-2 in humans and is expressed mainly in epithelial and tumor cells (21). Rhodocytin, on the other hand, is a platelet activating protein isolated from the Malayan pit viper *Calloselasma rhodostoma* (11). Also envisaged as activators of CLEC-2 are derivatives of podoplanin or rhodocytin, as long as the derivatives retain the biological function of these proteins, i.e. the ability to act as CLEC-2 ligands and to induce platelet activation.

Four transcript variants of human podoplanin are known, the mRNA sequences of which can be found e.g. under the NCBI accession numbers NM_006474.4 (transcript variant 1), NM_98389.2 (transcript variant 2). NM_001006624.1 (transcript variant 3), and NM_001006625.1 (transcript variant 4). Rhodocytin is expressed as two chains, namely chain A, (Protein Database PDB Accession number: 2VRP_A; GI:193885228) and chain B (Protein Database PDB Accession number: 2VRP_B; GI:193885229).

In a preferred embodiment of the pharmaceutical composition of the invention, the composition further comprises in the same or a separate container an antagonist of G-protein coupled receptors or signaling pathways.

As used herein, the term "antagonist of G-protein coupled receptors or signaling pathways" refers to any ligand or compound that does not provoke a biological response itself upon binding to a G-protein coupled receptors, but blocks or dampens agonist-mediated responses. Thus, the antagonist has affinity but no efficacy for G-protein coupled receptors. Binding of the antagonist results in disruption of the interaction and inhibits the function of the G-protein coupled receptor. Antagonists can mediate their effects by binding to the active site or to allosteric sites on the G-protein coupled receptor, or they may interact at unique binding sites not normally involved in the biological regulation of the receptor's activity. Depending on the nature of antagonist receptor binding, the antagonist activity may be reversible or irreversible depending on the longevity of the antagonist-receptor complex.

In a more preferred embodiment of the pharmaceutical composition, the inhibitor or the method of the invention, the antagonist of G-protein coupled receptors or signaling pathways is aspirin or is an inhibitor of P2Y1, P2Y12 or PAR receptors.

The term "aspirin", as used in accordance with the present invention, refers acetylsalicylic acid (ASA), which is a salicylate drug. Aspirin has an antiplatelet, i.e. an "anti-coagulation", effect by inhibiting thromboxane prostaglandins, which under normal circumstances effect platelet aggregation to repair damaged blood vessels. Thus, aspirin is used in low doses in medicine for the long-term prevention of heart attacks, strokes, and blood clot formation in people at high risk for developing blood clots.

In accordance with the present invention, the term "P2Y1 receptor" refers to a seven-transmembrane receptor for adenosine diphosphate (ADP) that couples to heterotrimeric G protein (Gq) in platelets.

The term "P2Y12 receptor", according to the present invention, refers to a seven-transmembrane receptor for adenosine diphosphate (ADP) that couples to heterotrimeric G protein (Gi) in platelets.

As used herein, the term "PAR receptor" refers to seven-tranamembrane protease activated receptors for thrombin (PAR1 and PAR4) that couple to heterotrimeric G proteins in platelets.

In a preferred embodiment of the inhibitor of the invention, the inhibitor has admixed thereto or associated in a separate container an antagonist of G-protein coupled receptors or signaling pathways. In another preferred embodiment of the inhibitor of the invention, the inhibitor has admixed thereto anti-coagulants which are known in the art, described for example in W02006/066878, which is incorporated herein in its entirety.

In a preferred embodiment of the method of the invention, the method further comprises the administration of an antagonist of G-protein coupled receptors or signaling pathways. In another preferred embodiment of the method of the invention, the method further comprises the administration of anti-coagulants which are known in the art, described for example, in WO2006/066878 which is specifically incorporated herein in its entirety.

In a further preferred embodiment of the inhibitor or the method of the invention, the disorder related to venous or arterial thrombus formation is selected from the group consisting of myocardial infarction, stroke, ischemic stroke, pulmonary thromboembolism, peripheral artery disease (PAD), arterial thrombosis and venous thrombosis.

The term "myocardial infarction", as used herein, relates to a medical condition generally referred to as "heart attack" and is characterized by interrupted blood supply to the heart. The resulting ischemia (oxygen shortage) causes cellular damage and - depending on the length of ischemia - even tissue necrosis. Most commonly, myocardial infarct is due to the rupture of a vulnerable plaque that leads to a blockade of a vein or artery.

The term "stroke" is well-known in the art and is sometimes also referred to as cerebrovascular accident (CVA). A stroke is a medical condition that is medically defined by reduced blood supply to the brain resulting in loss of brain function, inter alia due to ischemia. Said reduction in blood supply can be caused, for example, by thrombosis or embolism, or due to hemorrhage. Hence, strokes are generally classified into two major categories, ie., i) ischemic and ii) hemorrhagic strokes. Ischemia is due to an interruption in blood circulation and hemorrhage is due to a rupture of a blood vessel, both scenarios ultimately leading to a reduced blood supply of the brain. The prevalent form of stroke is the ischemic stroke accounting for about 80% of strokes. In an ischemic stroke, blood supply to part of the brain is decreased, leading to dysfunction and necrosis of the brain tissue in that area. There are mainly three causative reasons: thrombosis (obstruction of a blood vessel by a blood clot forming locally), embolism (idem due to a blood clot from elsewhere in the body) and systemic hypoperfusion (general decrease in blood supply, e.g. in shock).

The term "pulmonary thromboembolism", as used in accordance with the present invention, relates to a blockage of one of the arteries in the lung by a blood clot. Microscopic thrombi, or clots, grow under certain conditions to form a larger clot that blocks a vein. This is called deep vein thrombosis (DVT). DVTs usually form in the veins of the lower leg or pelvis, but can start anywhere in the body. Pieces may break loose and travel through the bloodstream to the lung, where they block the pulmonary blood vessels and stop blood travelling through the lung to collect oxygen. This is a pulmonary embolism (PE), the result of which is dangerously low levels of oxygen in the blood and tissues.

"Peripheral artery disease (PAD)", as used herein, relates to diseases caused by the obstruction of large arteries in the arms and legs. PAD can result from atherosclerosis, inflammatory processes leading to stenosis, an embolism or thrombus formation. It causes either acute or chronic ischemia (lack of blood supply), typically of the legs.

As used in accordance with the present invention, the term "arterial thrombosis" relates to the formation of a thrombus within an artery. In most cases, arterial thrombosis follows rupture of atheroma, and is therefore referred to as atherothrombosis. There are two major diseases which can be classified under this category: stroke and myorcardial infarction.

The term "venous thrombosis", according to the present invention, relates to blood clot formation within a vein. Superficial venous thromboses can cause discomfort but generally do not cause serious consequences, unlike the deep vein thromboses (DVTs) that form in the deep veins of the legs or in the pelvic veins. Since the veins return blood to the heart, breaking off of a piece of a blood clot formed in a vein can lead to its being transported to the right side of the heart, and from there into the lungs, leading to pulmonary embolism as described above.

All of the diseases described herein are well known to the skilled person and are defined in accordance with the prior art and the common general knowledge of the skilled person.

In a more preferred embodiment of the inhibitor or the method of the invention, the stroke is ischemic stroke.

The present invention also relates to a method of identifying a compound suitable as a lead compound and/or as a medicament for the treatment and/or prevention of a disorder related to venous or arterial thrombus formation, comprising the steps of (a) determining the level of CLEC-2 protein or *Clec*-2 transcript in thrombocytes; (b) contacting said thrombocytes or thrombocytes of the same population with a test compound; (c) determining the level of CLEC-2 protein or *Clec*-2 transcript in said thrombocytes after contacting with the test compound; and (d) comparing the level of CLEC-2 protein or *Clec*-2 transcript determined in step (c) with the CLEC-2 protein or *Clec*-2 transcript level determined in step (a), wherein a decrease of CLEC-2 protein or *Clec*-2 transcript level in step (c) as compared to step (a) indicates that the test compound is a compound suitable as a lead compound and/or as a medicament for the treatment and/or prevention of a disorder related to venous or arterial thrombus formation.

This embodiment relates to a cellular screen, wherein inhibitors may be identified which exert their inhibitory activity by interfering with the expression of CLEC-2, either by affecting the stability of CLEC-2 protein or transcript (mRNA) or by interfering with the transcription or translation of CLEC-2.

A "compound" in accordance with the present invention can be any of the inhibitors defined above, i.e. an antibody or a fragment or derivative thereof, an aptamer, a siRNA, a shRNA, a miRNA, a ribozyme, an antisense nucleic acid molecule or a small molecule. Test compounds further include but are not limited to, for example, peptides such as soluble peptide, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D- and/or L-configuration amino acids or phosphopeptides (e.g., members of random and partially degenerate, directed phosphopeptide libraries, see, e.g., Songyang et al. (1993) Cell 72: 767-778).

The term "thrombocytes or thrombocytes of the same population" as used herein refers either to the thrombocytes used in step (a) or to thrombocytes being of the same origin as the thrombocytes of step (a) and having identical characteristics to the thrombocytes of (a). Furthermore, this term both encompasses thrombocyte populations, such as for example homogenous cell populations consisting of cells having identical characteristics, as well as single thrombocytes for single cell analyses.

As described hereinabove, CLEC-2 is an is an essential mediator of platelet activation *in vitro* and *in vivo.* Therefore, the use of CLEC-2 as a target for the discovery of compounds that are suitable for the treatment and/or prevention of a disorder related to venous or arterial thrombus formation is also encompassed by the present invention. It is envisaged that a decrease of expression levels of CLEC-2 conferred by a compound as described above may contribute to protection from venous or arterial thrombus formation and may ameliorate conditions associated therewith, as described above. Accordingly, measurement of the CLEC-2 protein or *CLEC*-2 transcript level may be used to determine the readout of the above-described assay.

For example, the above-mentioned thrombocytes may exhibit a detectable level of CLEC-2 protein or CLEC-2 transcript before contacting with the test compound and the level of CLEC-2 protein or CLEC-2 transcript may be lower or undetectable after contacting the thrombocytes with the test compound, indicating a compound suitable for the treatment and/or prevention of a disorder related to venous or arterial thrombus formation or as a lead compound for the development of a compound for this treatment. Preferably, the level of CLEC-2 protein or *CLEC-2* transcript after contacting the thrombocytes with the test compound is reduced by, for example, at least 10, 20, 30, 40 or 50% as compared to the level of CLEC-2 protein or *CLEC-2* transcript before contacting the thrombocytes with the test compound. More preferably, the level of CLEC-2 protein or *CLEC-2* transcript after contacting the thrombocytes with the test compound is reduced by, for example, at least 60, 70, 80, 90 or 95% as compared to the level of CLEC-2 protein or *CLEC-2* transcript before contacting the thrombocytes with the test compound. Most preferably, the level of CLEC-2 protein or *CLEC-2* transcript after contacting the thrombocytes with the test compound is reduced by 100% as compared to the level of CLEC-2 protein or *CLEC-2* transcript before contacting the thrombocytes with the test compound. The term "the level of CLEC-2 protein or *CLEC-2* transcript is reduced by [...] %" refers to a relative decrease compared to the level of CLEC-2 protein or *CLEC-2* transcript before contacting the thrombocytes with the test compound. For example, a reduction of at least 40% means that after contacting the thrombocytes with the test compound the remaining level of CLEC-2 protein or CLEC-2 transcript is only 60% or less as compared to the level of CLEC-2 protein or *CLEC-2* transcript before contacting the thrombocytes with the test compound. A reduction by 100% means that no detectable level of CLEC-2 protein or CLEC-2 transcript remains after contacting the thrombocytes with the test compound.

Measurements of protein levels as well as of transcript level can be accomplished in several ways, as described above.

The present invention further relates to a method of identifying a compound suitable as a lead compound and/or as a medicament for the treatment and/or prevention of a disorder related to venous or arterial thrombus formation, comprising the steps of (a) determining the level of CLEC-2-mediated activation of thrombocytes upon stimulation of the thrombocytes with a CLEC-2 ligand; (b) contacting said thrombocytes or thrombocytes of the same population with a test compound; (c) determining the level of CLEC-2-mediated activation of thrombocytes upon stimulation of the thrombocytes with a CLEC-2 ligand after contacting with the test compound; and (d) comparing the level of CLEC-2-mediated activation of thrombocytes upon stimulation of the thrombocytes with a CLEC-2 ligand determined in step (c) with the level of CLEC-2-mediated activation of thrombocytes upon stimulation of the thrombocytes with a CLEC-2 ligand determined in step (a), wherein a decrease in the level of CLEC-2-mediated activation of thrombocytes in step (c) as compared to step (a) indicates that the test compound is a compound suitable as a lead compound and/or as a medicament for the treatment and/or prevention of a disorder related to venous or arterial thrombus formation.

This embodiment relates to a cellular screen, wherein inhibitors may be identified which exert their inhibitory activity by physically interacting with CLEC-2, or alternatively (or additionally) by functionally interacting with CLEC-2, i.e., by interfering with the pathway(s) present in the thrombocytes employed in the cellular assay. As a result, the biological activity of CLEC-2 is altered either directly or indirectly.

This method includes as a first step the stimulation of thrombocytes with a CLEC-2 ligand and the determination of the level of CLEC-2-mediated activation of the thrombocytes. In the next step of the method, the thrombocytes or thrombocytes of the same population are contacted with a test compound and, in a third step, the level of CLEC-2-mediated activation of thrombocytes upon stimulation of the thrombocytes with a CLEC-2 ligand after contacting with the test compound is determined. The thus observed level of CLEC-2-mediated activation of thrombocytes is compared between the first step carried out in the absence of the test compound and the third step, carried out after contacting with the test compound. If after contacting with the test compound a reduction in CLEC-2-mediated activation of thrombocytes is observed, then the test compound is an inhibitor of CLEC-2-mediated activation and is thus suitable as a lead compound and/or as a medicament for the treatment and/or prevention of a of a disorder related to venous or arterial thrombus formation. Most preferably, the test compound results in no CLEC-2-mediated activation of thrombocytes at all, i.e. the test compound successfully inhibits CLEC-2-mediated activation completely, i.e. to 100%.

In a preferred embodiment, the activation of thrombocytes is determined by measuring the amount of thrombocyte aggregation, by determining the amount of fibrinogen binding, by measuring the amount of surface exposure of P-selectin and/or by measuring thrombus growth on a collagen-coated surface under flow conditions. These methods are known in the art and/or are described in detail in the examples below.

In a preferred embodiment of this method of the invention, the CLEC-2 ligand is podoplanin or rhodocytin.

In a preferred embodiment, the methods of the invention are carried out *in vitro.*

*In vitro* methods offer the possibility of establishing high-throughput assays, as described above.

The identified so-called lead compounds may further be optimized to arrive at a compound which may be used in a pharmaceutical composition. Methods for the optimization of the pharmacological properties of compounds identified in screens, the lead compounds, are known in the art and comprise, for example, the methods described above for the modifications of inhibitors.

### The figures show:

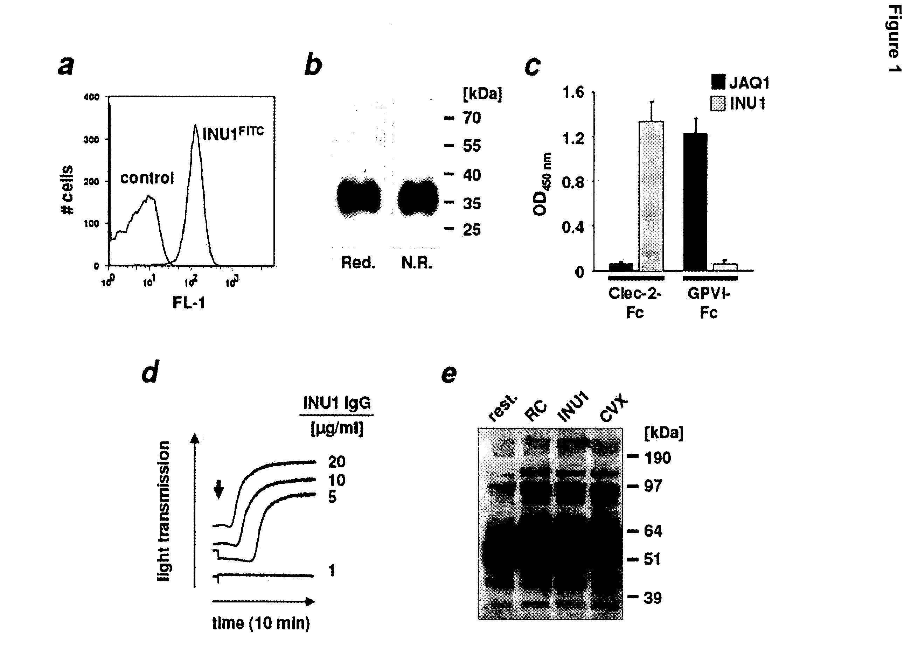

**Figure 1****: INU1 recognizes murine CLEC-2.** (A) Flow cytometric detection of CLEC-2 on mouse platelets. Platelets were incubated with INU1-FITC (solid line) or an irrelevant rat IgG-FITC (shaded area) at saturating concentrations for 15 min at RT and analyzed directly. (B) Immunoprecipitation of CLEC-2 from surface-biotinylated mouse platelets. NP-40 lysates were incubated with 10 µg/ml INU1, followed by protein G-Sepharose. Proteins were separated on a 12% SDS-PAGE gel under reducing (red.) or nonreducing (n.r.) conditions, transferred onto a PVDF membrane, and detected by streptavidin-HRP and ECL. (C) Binding of INU1 to mCLEC-2-Fc or mGPVI-Fc was tested by ELISA, The anti-mouse GPVI antibody, JAQ1, was used as a control (n=3). (D) Washed platelets from control mice were incubated with the indicated concentrations of INU1 and light transmission was recorded on a Fibrintimer 4 channel aggregometer. The results are representative of 4 individual experiments. (E) Washed platelets were stimulated with vehicle (rest.), 0.24 µg/ml rhodocytin (RC), 20 µg/ml INU1, or 1 µg/ml convulxin (CVX), lysed after 90 sec and probed with the phosphotyrosine-specific antibody 4G10 and ECL.
**Figure 2****: INU1-Fab partially inhibits CLEC-2 function *in vitro.*** (A) INU1-Fab (20 µg/ml) only induces aggregation of washed control platelets when crosslinked (20 µg/ml anti-rat IgG). (B, C) Washed control platelets were incubated with vehicle (black) or INU1-Fab (20 µg/ml, gray) for 5 min at 37°C and then stimulated with the indicated agonists under stirring conditions. Light transmission was recorded on a Fibrintimer 4 channel aggregometer. The results are representative of 4 individual measurements.
**Figure 3****: INUI induces the loss of CLEC-2 in circulating platelets *in vivo.*** (A) Mice received the indicated amounts of purified IgG or Fab fragments of INU1 i.v. in 200 µl sterile PBS and platelet counts were determined by flow cytometry at the indicated times. Results are expressed as the mean platelet count ± SD for groups of each 4 mice and are representative of 3 individual experiments, Parallel analyses on an automated cell analyser (Sysmex) yielded similar results (not shown). (B) Two color flow cytometric analysis of integrin αIIbβ3 activation (JON/APE) and P-selectin exposure (FITC) in platelets from mice 1 or 3 days after INU1-IgG or INU1-Fab injection. Diluted whole blood was stimulated with 1 µg/ml rhodocytin (RC). The data are representative of 8 mice per group. (C) Mice were injected with the indicated amounts INU1-IgG or INU1-Fab and surface-bound IgG or Fab was detected by flow cytometry using anti-rat IgG-FITC. Maximal binding was determined by incubating platelets from untreated mice with INU1-IgG or INU1-Fab (10 µg/ml) *in vitro.* (D) INU1-FITC binding to platelets from the indicated mice. (E) Immunoprecipitation of CLEC-2 and GPVI from surface-biotinylated platelets from control and INU1 (8 µg/g)-treated mice on day 5 under reducing conditions.
**Figure 4****: CLEC-2-deficient platelets show abolished response to rhodocytin but normal responses to classic agonists.** Mice were treated with vehicle or 8 µg/g INU1 and analysed on day 5. (A) Washed platelets from control (black) or INU1-treated (gray) mice were stimulated with the indicated agonists and light transmission was recorded on a Fibrintimer 4 channel aggregometer. The results are representative of 6 individual experiments. (B) Flow cytometric analysis of αIIbβ3 integrin activation (binding of JON/A-PE, left panel) and degranulation-dependent P-selectin exposure (right panel) in response to the indicated agonists from control (black) or INU1-treated (gray) mice. Abbreviation: U46: U46619. Results are mean fluorescent intensities (MFI) ± SD of 6 mice per group.
**Figure 5****: CLEC-2-deficient platelets fail to form stable aggregates under flow.** Whole blood from control or INU1-treated mice (8 µg/g, day 5) was perfused over a collagen-coated surface (0.2 mg/ml) at a shear rate of 1,700s⁻¹. (A) Platelet adhesion on collagen after 30 s, indicated as number of platelets per visual field. (B) Aggregate formation on collagen after 4 min perfusion time. Top: representative phase contrast images. Bar, 100 µm. Bottom: Mean surface coverage (left) and relative thrombus volume expressed as integrated fluorescence intensity (IFI) per mm² (right) ± SD of 6 mice per group, ^{**}*p*<0.01.
**Figure 6****: Defective thrombus formation and prolonged bleeding times in CLEC-2-deficient mice.** Mice received vehicle or INU1 (8 µg/g) and were analysed after 5 days. (A-C) Mesenteric arterioles were injured with FeCl₃ and adhesion and thrombus formation of fluorescently-labeled platelets was monitored *in vivo* by fluorescence microscopy. (A) Time to appearance of first thrombus >10 min, and (B) time to vessel occlusion are shown. Each symbol represents one individual. (C) Representative images are depicted. Bar, 50 µm. Each symbol represents one individual. The asterisk indicates occlusion of the vessel. (D) Tail bleeding times in control and CLEC-2-deficient mice. Each symbol represents one individual.

The examples illustrate the invention.

### Example 1: Materials and methods

***Mice**.* Specific-pathogen-free male mice (NMRI) 4 to 10 weeks of age were obtained from Harlan Winkelmann (Borchen, Germany) and kept in our animal facilities. Animal studies were approved by the district government of Lower Franconia (Bezirksregierung Unterfranken).

***Chemicals and antibodies.*** Anesthetic drugs: medetomidine (Pfizer, Karlsruhe, Germany), midazolam (Roche Pharma AG, Grenzach-Wyhlen, Germany), fentanyl (Janssen-Cilag GmbH, Neuss, Germany) and antagonists: atipamezol (Pfizer, Karlsruhe, Germany), flumazenil and naloxon (both from Delta Select GmbH, Dreieich, Germany) were used according to the regulation of the local authorities. ADP (Sigma, Deisenhofen, Germany), U46619 (Alexis Biochemicals, San Diego, USA), thrombin (Roche Diagnostics, Mannheim, Germany), and collagen (Kollagenreagents Horm, Nycomed, Munich, Germany) were purchased. Mouse α-rat IgG (Dianova, Hamburg, Germany) and α-rat FITC (DAKOCytomation, Denmark) were purchased. Rhodocytin was isolated as described (18). Monoclonal antibodies conjugated to fluorescein isothiocyanate (FITC) or phycoerythrin (PE), or DyLight-488 were from Emfret Analytics (Würzburg, Germany). The JON/A-PE antibody preferentially binds to the high affinity conformation of mouse αIIbβ3 integrin (43).

***Production of monoclonal antibodies (mAbs).*** Female Wistar rats, 6-8 weeks of age, were immunized repeatedly with mouse platelets. The rat spleen cells were then fused with mouse myeloma cells (Ag14.653) and hybridomas were selected in HAT medium. Hybridomas secreting mAbs directed against platelet receptors were identified by flow cytometry. Briefly, a 1:1 mixture of resting and thrombin-activated platelets (10⁶ cells) was incubated with 100 µL, supernatant for 30 min at room temperature, washed with PBS (1,300g, 10 min) and stained with FITC-labeled rabbit anti-rat Ig for 15 min. The samples were analyzed on a FACScalibur (Becton Dickinson, Heidelberg, Germany) in the set up mode. Platelets were gated by FSC/SSC-characteristies. Positive hybridomas were subcloned twice prior to large scale production.

***Modification of antibodies.*** Fab fragments from INU1 were generated by 12-hour incubation of 10 mg/mL mAb with immobilized papain (Pierce), and the preparations were then applied to an immobilized protein A column followed by an immobilized protein G column (Biorad, Munich, Germany and GEHealtheare, Sweden) to remove Fc fragments and any undigested IgG. The purity of the Fab fragments was checked by sodium dodecyl sulphate-polyacrylamide gel electrophoresis (SDS-PAGE).

***mCLEC-2-Fc fusion protein.*** For cloning of extracellular domain of mouse CLEC-2, RNA from mouse bone marrow was isolated using the RNeasy Mini Kit (Qiagen) according to the manufactures protocol and reverse transcription was performed (Titan One Tube RT-PCR-Kit, Roche, Ingelheim, Germany). 100 ng RNA was used as the template and the oligonucleotide AA CTC GAG ACA CAG CAA AAG TAT CTA (with the Xhol site underlined) and AA GGATC C AGC AGT TTG TCC ACT CTT (with the *Bam HI* site underlined) as the forward and reverse primer, respectively. The PCR-product was purified using a QIAquick gel extraction kit (Qiagen, Hilden, Germany) digested with *Xho*I and *Bam*HI, purified again and ligated to the Signal pig plus vector containing the human immunoglobulin Fc-domain. The ligation mixture was transformed into *E*. *coli* DH5□. The obtained construct was verified by restriction enzyme digestion and DNA-sequencing. Human embryonic kidney (HEK) 293 cells were transfected with the construct using the calcium phosphate method. Stable cell lines expressing recombinant mClec-2-Fc were selected in medium containing 700 µg/mL geneticin. The mCLEC-2-Fc protein was purified on a protein-G-sepharose column (26).

***Immunoprecipitation.*** Washed platelets (10⁸) were surface labeled with EZ-Link sulfo-NHS-LC-biotin (Pierce, 100 µg/mL in PBS) and subsequently solubilized in 1 mL lysis buffer (Tris-buffered saline containing 20 mmol/L Tris/HCl, pH8.0, 150 mmol/L NaCl, 1 mmol/L EDTA, 1 mmol/L phenylmethylsulfonyl fluoride, 2 µg/mL aprotinin, 0.5 µg/mL leupeptin and 0.5% Nonidet P-40 - all from Boehringer Mannheim, Germany). Cell debris was removed by centrifugation (15,000g, 10 min). Following preclearing (8 h), 10 µg mAb was added along with 25 µL of protein G-Sepharose and precipitation took place overnight at 4°C. Samples were separated on a 12 % SDS-polyacrylamide gel (PAGE) along with a molecular weight marker and transferred onto a polyvinylidene difluoride (PVDF) membrane. The membrane was incubated with streptavidin-horseradish peroxidase (1 µg/ml) for 1 h after blocking. After extensive washing, biotinylated proteins were visualized by ECL (Amersham, GEHealthcare).

***Platelet aggregation and flow cytometry.*** Platelet aggregation: Washed platelets (200 µl with 0.5 x 10⁶ platelets/µl) were analyzed in the presence of 70 µg/ml human fibrinogen. Transmission was recorded on a Fibrintimer 4 channel aggregometer (APACT Laborgeräte und Analysensysteme, Hamburg, Germany) over ten minutes, and was expressed in arbitrary units with buffer representing 100% transmission. Flow cytometry: Washed blood was diluted 1:20 and incubated with appropriate fluorophore-conjugated monoclonal antibodies for 15 min at RT and analyzed on a FACScalibur instrument (Becton Dickinson, Heidelberg, Germany).

***Adhesion under flow conditions.*** Rectangular coverslips (24 x 60 mm) were coated with 0.2 mg/ml fibrillar type I collagen (Nycomed, Munich, Germany) for 1 h at 37°C and blocked with 1% BSA. Heparinized whole blood was labeled with a Dylight-488 conjugated anti-CPIX Ig derivative at 0.2 µg/ml and perfusion was performed as described (28). Image analysis was performed off-line using Metavue software (Visitron, Munich, Germany). Thrombus formation was expressed as the mean percentage of total area covered by thrombi, and as the mean integrated fluorescence intensity per mm² as described (36).

***Bleeding time.*** Mice were anesthetized and a 1 mm segment of the tail tip was removed with a scalpel. Tail bleeding was monitored by gently absorbing blood with filter paper at 20 second intervals, without making contact with the wound site. When no blood was observed on the paper, bleeding was determined to have ceased. Experiments were stopped after 20 minutes.

***Intravital microscopy of thrombus formation in FeCl₃*-*injured mesenteric arterioles.*** Mice (4-5 weeks of age) were anesthetized, and the mesentery was exteriorized through a midline abdominal incision. Arterioles (35-60 µm diameter) were visualized with a Zeiss Axiovert 200 inverted microscope (x10) equipped with a 100-W HBO fluorescent lamp source, and a CooISNAP-EZ camera (Visitron, Munich Germany). Digital images were recorded and analyzed off-line using Metavue software. Injury was induced by topical application of a 3 mm² filter paper saturated with FeCl₃ (20%). Adhesion and aggregation of fluorescently labeled platelets (Dylight-488 conjugated anti-GPIX Ig derivative) in arterioles was monitored for 40 min or until complete occlusion occurred (blood flow stopped for >1 min).

***Statistics.*** Results from at least three experiments per group are presented as mean ± SD. Differences between control and INU1-treated groups were assessed by the Mann-Whitney U-test.. P-values < 0.05 were considered statistically significant.

***Abbreviations used:*** mAb, monoclonal antibody; CVX, convulxin; FcR, Fc receptor; ITAM, immunoreceptor tyrosine-based activation motif; vWF, von Willebrand factor.

### Example 2: Monoclonal antibody generation against mouse CLEC-2

A new rat monoclonal antibody (mAb) against mouse CLEC-2 (INU1, rat IgG1) was generated. INU1 bound to mouse platelets (Fig. 1A) and precipitated a protein of an apparent molecular weight of approximately 32-38 kD under reducing and nonreducing conditions (Fig. 1B), demonstrating that the apparent molecular weight of mouse CLEC-2 is similar to its human homologue (11;25). The antigenic specificity of INU1 for CLEC-2 was confirmed by ELISA where it bound to the extracellular domain of mouse CLEC-2 fused to the Fc-portion of human IgG1 (mCLEC-2-Fc), but not to mGPVI-Fc (26). In contrast, the anti-mouse GPVI antibody, JAQ1 (27), bound to mGPVI-Fc, but not to mCLEC-2-Fc (Fig. 1C). INU1 did not recognize CLEC-2 in a Western blot analysis of platelet lysates, indicating that the antibody binds to a three-dimensional epitope on the receptor that is lost under the denaturing conditions during SDS-PAGE (not shown).

In line with previous observations with (polyclonal) anti-CLEC-2 antibodies in human platelets (11), INU1 induced aggregation of mouse platelets (Fig. 1D). This response occurred in a dose-dependent manner evident as a decrease in the delay before the onset of aggegation rather than an increase in the maximal aggregation response. A similar effect can be seen with rhodocytin on human platelets (11) and mouse platelets (not shown). INU1-induced activation was associated with changes in the tyrosine phosphorylation patterns that were comparable to those induced by rhodocytin (Fig. 1 E).

In contrast to INU1-IgG, monovalent Fab fragments of the antibody did not induce aggregation at concentrations up to 20 µg/ml (Fig. 2A), suggesting that receptor dimerization /clustering is a critical prerequisite for CLEC-2-mediated platelet activation. This was confirmed by crosslinking of the bound Fab fragments by a secondary antibody (rabbit anti-rat IgG) which induced robust aggregation (Fig. 2A). INU1-Fab (20 µg/ml) had no effect on the aggregation response to the stable TxA₂ analog U46619, the GPVI agonist convulxin (CVX) or thrombin, but ADP-induced aggregation was consistently increased in the presence of the antibody fragment (83.53 ± 4.57 % vs. 58.76 ± 5.12 % in control) (Fig. 2B). In contrast, INU1-Fab had a significant inhibitory effect on rhodocytin-induced platelet aggregation at low and intermediate agonist concentrations (visible as a delay in the onset of aggregation) which was, however, overcome at high rhodocytin concentrations (Fig. 2C). These results suggested that INU1 binds to an epitope on CLEC-2 that is at least partially overlapping with the rhodocytin binding site on the receptor.

Monovalent INU1-Fab fragments amplified ADP-induced aggregation indicating that occupancy of the INU1 binding epitope on CLEC-2 induces subliminal signaling independently of receptor clustering. This notion may also be supported by the observation that INU1-Fab induced transient thrombocytopenia to the same extent as the intact IgG. It is not clear whether the transient drop in platelet counts is mechanistically linked to the loss of CLEC-2, but it is feasible to speculate that INU1-opsonized platelets might become (partially) activated and transiently trapped in the reticulo-endothelial system where the actual loss of CLEC-2 occurs. In the case of JAQ-induced GPVI downregulation, shedding of the receptor is associated with transient thrombocytopenia, whereas internalization/degradation can occur independently thereof (33).

### Example 3: INU1-induced loss of CLEC-2 in circulating platelets

To study the effect of INU1 on platelets *in vivo,* mice received 2 µg/g body weight of the antibody i.v. and circulating platelets were studied *ex vivo* at different time points after injection. INU1 treatment induced transient thrombocytopenia with a maximum drop of platelet counts >85 % on day 1 and a return to normal or in some animals slightly increased platelet counts after 3-4 days where they remained for at least 6 more days (Fig. 3A). This effect was not Fc-dependent or caused by antibody-induced receptor dimerization as it was also seen with Fab fragments of INU1 (2 µg/g body weight) (Fig. 3A). INU1 treated mice did not develop spontaneous bleeding for at least three weeks (not shown).

On day 1 and 3, platelets from INU1-IgG or INU1-Fab treated mice were refractory towards rhodocytin (1 µg/ml) as revealed by flow cytometry (Fig. 3B), whereas the response was partially restored on day 5 and back to normal on day 7 (not shown). Notably, this inhibitory effect was not due to blockade of the receptor by INU1, as the antibody (or Fab fragment) was not detectable by a FITC-conjugated anti-rat IgG antibody on the surface of the cells on day 1 and 3 after injection whereas control platelets incubated with INU1-IgG or INU1-Fab *in vitro* yielded strong signals (Fig. 3C). INU1-FITC did not bind to platelets from INU1-IgG or INU1-Fab treated mice on day 1 and 3 whereas a subpopulation of the cells was stained on day 5 and >90 % on day 6 (Fig. 3D). These results indicated that INU1 treatment induced the loss of CLEC-2 in circulating platelets within 24 hours and that this effect was overcome by the production of new platelets.

To study the functional consequences of CLEC-2 deficiency in more detail and to test whether a prolonged loss of the receptor can be achieved, mice received 8 µg/g body weight INU1 i.v.. The resulting thrombocytopenia and the kinetics of CLEC-2 loss were comparable to the effects caused by 2 µg/g body weight (Fig. 3A) but CLEC-2 was not detectable on the surface of the cells for at least 6 days (Fig. 3C,D). Immunoprecipitation experiments performed on day 5 after antibody injection confirmed the complete loss of CLEC-2 whereas other receptors such as GPVI were not affected (Fig. 3E). This was also shown by flow cytometric analysis of basal surface expression levels of GPVI, GPIb-V-IX, CD9 and integrins αIIbβ3 and α2β1 on day 5 (table 1). Hematocrits and white blood cell counts were not significantly different between control and INU1-treated mice (table 1).

Standard aggregometry revealed that platelets from mice on day 5 after INU1 injection (8 µg/g body weight) were completely resistant to activation with rhodocytin at any concentration tested (up to 10 µg/ml) whereas responses to ADP, U46619, CVX, collagen and thrombin were normal (Fig. 4A). Flow cytometric analyses confirmed that INU1-induced CLEC-2 deficiency had no significant effect on platelet activation by ADP, U46619, CVX or thrombin, whereas responses to rhodocytin were blunted (Fig. 4B). On day 10 after INU1 injection, CLEC-2 expression (Fig. 3D) and rhodocytin-induced responses (not shown) were fully restored in all tested animals, Together, these results demonstrated that INU1-induced CLEC-2 deficiency very specifically abolished one activation pathway in platelets while leaving other ones fully intact.

**Table 1. Hematology and platelet glycoprotein expression in control and INU1-treated mice**

| | **control** | **INU1** |
|---|---|---|
| **HCT [%]** | 53.3 ± 1.76 | 48.7 ± 4.41 |
| **WBC** | 11.1 ± 1.19 | 11.05 ± 1.74 |
| **[x10⁶]** | | |
| **Clec-2** | 119 ± 10 | 14 ± 1 |
| **GPIb** | 398 ± 5 | 391 ± 19 |
| **GPV** | 354 ± 5 | 364 ± 14 |
| **GPIX** | 550 ± 13 | 563 ± 20 |
| **CD9** | 1496 ± 27 | 1338 ± 66 |
| **GPVI** | 61 ± 3 | 56 ± 3 |
| **α2** | 76 ± 5 | 69 ± 1 |
| **β1** | 173 ± 11 | 165 ± 12 |
| **αIIbβ3** | 888 ± 40 | 854 ± 51 |

Hematocrit (HCT) in per cent and white blood cell counts (WBC) per ml for control and INU1-treated mice (day 5) were analyzed with an automated blood analyzer (Sysmex, n=6 mice per group). Expression of glyocoproteins on the platelet surface was determined by flow cytometry. Diluted whole blood from the indicated mice was incubated with FITC-labeled antibodies at saturating concentrations for 15 min at RT and platelets were analyzed directly. Results are expressed as mean fluorescence intensity ± SD for 6 mice per group.

Although it was not possible to identify the route of CLEC-2 downregulation, mClec-2A expressed in HEK293T cells has been shown to be proteolytically cleaved to produce a soluble extracellular fragment (32). This indicates that the loss of CLEC-2 could occur through ectodomain shedding, but also internaiization/degradation might contribute to this process. We have previously demonstrated that antibody-induced downregulation of GPVI can occur through both of these routes and that these are regulated by diverging signaling pathways downstream of the receptor in vivo (33). These are, however, difficult to assess as neither GPVI (30;31;34;35) nor CLEC-2 (not shown) can be downregulated by antibodies in vitro. This indicates that additional signals or a certain environment may be required for this process to occur that is absent in our in vitro assays. It is clear, however, that the Fc part of the antibody or receptor dimerization is not required for CLEC-2 downregulation in vivo as Fab fragments are equally efficient compared to the intact IgG.

CLEC-2-deficient humans have not been reported to date and it is not clear whether mutations in the CLEC-2 gene affect development in humans or mice. However, it can be anticipated that patients with an acquired CLEC-2 deficiency exist, caused by autoantibody-induced CLEC-2 clearing in platelets as previously reported for GPVI-deficiencies (31;42). The identification of such patients might help to define the role of CLEC-2 in the physiology of human platelets.

### Example 4: CLEC-2 is required for stable thrombus formation under flow

At sites of vascular injury, the signals generated by multiple platelet receptor-ligand interactions are integrated to ensure efficient platelet attachment and thrombus formation under flow conditions (7). A possible function of CLEC-2 in this process has not been assessed to date. Therefore, we analyzed the ability of CLEC-2-deficient platelets to form thrombi on collagen-coated surfaces in a whole blood perfusion system (28). Under high shear conditions (1,700 s⁻¹), control platelets adhered to collagen fibers and formed aggregates within 2 min that consistently grew into large thrombi by the end of the perfusion period of 4 min. In marked contrast, while CLEC-2-deficient platelets exhibited unaltered adhesion to collagen under flow (Fig. 5A), the subsequent formation of three-dimensional aggregates was severely impaired (Fig. 5B). During the entire perfusion time, adherent platelets recruited numerous new platelets from the blood flow, but these were consistently unable to firmly attach and were released after a few seconds. As a consequence, the surface area covered by platelets and the total thrombus volume at the end of the experiment were reduced by 36% and 82%, respectively (Fig. 5B). Similar results were obtained at intermediate shear rates (1,000 s⁻¹ - not shown). These findings demonstrated that CLEC-2-dependent processes are essential for stable aggregate formation under flow, whereas the receptor is not required for the adhesion process on collagen.

### Example 5: Increased bleeding times and defective arterial thrombus formation in CLEC-2-deficient mice

As platelet aggregation is a major pathomechanism in acute ischemic cardiovascular events, we studied the effects of CLEC-2 deficiency on pathologic occlusive thrombus formation by *in vivo* fluorescence microscopy following ferric chloride-induced mesenteric arteriole injury. In all control mice, the formation of small aggregates was observed ∼5-7 minutes after injury, with progression to complete vessel occlusion within 20 min (mean occlusion time: 16.4 ± 2.2 min) (Fig. 6A-C). In contrast, while the initial adhesion and formation of small aggregates occurred with similar kinetics in CLEC-2-deficient mice (6.9 ± 1.5 min vs. 6.5 ± 1.5 min in control, *p*>0.05) progression to stable large thrombi was almost completely abrogated. This defect was to a great extent caused by the release of individual platelets from the thrombus surface but also embolization of small thrombus fragments was observed (not shown). Consequently, blood flow was maintained throughout the 40 min observation period in all CLEC-2-deficient mice, revealing a crucial role for CLEC-2 during occlusive thrombus formation.

To test whether the INU1-induced CLEC-2 deficiency has an impact on hemostasis, we measured tail bleeding times. While bleeding stopped in all (22/22) control mice during the 20 min observation period (mean bleeding time: 6.1 ± 3.9 min), bleeding times were increased in INU1-treated mice, with 8 of 24 (33.3 %) mice bleeding for >20 min and a mean bleeding time of 10.8 ± 6.0 min for the other animals (*p*<0.05) (Fig. 6D). These results show that CLEC-2 is required for normal hemostasis.

### References

1. Weiss,H.J. 1975. Platelet physiology and abnormalities of platelet function (second of two parts). N Engl J Med 293:580-588.
2. Ruggeri,Z.M. 2002. Platelets in atherothrombosis. Nat Med 8:1227-1234.
3. Conti,C.R. and J.L.Mehta. 1987. Acute myocardial ischemia: role of atherosclerosis, thrombosis, platelet activation, coronary vasospasm, and altered arachidonic acid metabolism. Circulation 75:V84-V95.
4. Bhatt,D.L. and E.J.Topol. 2003. Scientific and therapeutic advances in antiplatelet therapy. Nat.Rev.Drug Discov. 2:15-28.
5. Savage,B., F.Almus-Jacobs, and Z.M.Ruggeri. 1998. Specific synergy of multiple substrate-receptor interactions in platelet thrombus formation under flow. Cell 94:657-666.
6. Varga-Szabo,D., I.Pleines, and B.Nieswandt. 2008. Cell adhesion mechanisms in platelets. Arterioscler.Thromb.Vasc.Biol. 28:403-412.
7. Jackson,S.P., W.S.Nesbitt, and S.Kulkarni. 2003. Signaling events underlying thrombus formation. J Thromb Haemost 1:1602-1612.
8. Offermanns,S. 2006. Activation of platelet function through G protein-coupled receptors. Circ.Res. 99:1293-1304.
9. Watson,S.P., N.Asazuma, B.Atkinson, O.Berlanga, D.Best, R.Bobe, G.Jarvis, S.Marshall, D.Snell, M.Stafford, D.Tulasne, J.Wilde, P.Wonerow, and J.Frampton. 2001. The role of ITAM- and ITIM-coupled receptors in platelet activation by collagen. Thromb Haemost 86:276-288.
10. Nieswandt,B. and S,P.Watson. 2003. Platelet-collagen interaction: is GPVI the central receptor? Blood 102:449-481.
11. Suzuki-Inoue,K., G.L.Fuller, A.Garcia, J.A.Eble, S.Pohlmann, O.Inoue, T.K.Gartner, S.C.Hughan, A.C.Pearce, G.D.Laing, R.D.Theakston, E.Schweighoffer, N.Zitzmann, T.Morita, V.L.Tybulewicz, Y.Ozaki, and S.P.Watson. 2006. A novel Syk-dependent mechanism of platelet activation by the C-type lectin receptor CLEC-2. Blood 107:542-549.
12. Fuller,G.L., J.A.Williams, M.G.Tomlinson, J.A.Eble, S.L.Hanna, S.Pohlmann, K.Suzuki-Inoue, Y.Ozaki, S.P.Watson, and A.C.Pearce. 2007. The C-type lectin receptors CLEC-2 and Dectin-1, but not DC-SIGN, signal via a novel YXXL-dependent signaling cascade. J.Biol.Chem. 282:12397-12409.
13. Colonna,M., J.Samaridis, and L.Angman. 2000. Molecular characterization of two novel C-type lectin-like receptors, one of which is selectively expressed in human dendritic cells. Eur.J.Immunol. 30:697-704.
14. Sobanov,Y., A.Bemreiter, S.Derdak, D.Mechtcheriakova, B.Schweighofer, M.Duchler, F.Kalthoff, and E.Hofer. 2001. A novel cluster of lectin-like receptor genes expressed in monocytic, dendritic and endothelial cells maps close to the NK receptor genes in the human NK gene complex. Eur.J.Immunol, 31:3493-3503.
15. Navdaev.A., J.M.Clemetson, J.Polgar, B.E.Kehrel, M.Glauner, E.Magnenat, T.N.Wells, and K.J.Clemetson. 2001. Aggretin, a heterodimeric C-type lectin from Calloselasma rhodostoma (malayan pit viper), stimulates platelets by binding to alpha 2beta 1 integrin and glycoprotein Ib, activating Syk and phospholipase Cgamma 2, but does not involve the glycoprotein VI/Fc receptor gamma chain collagen receptor. J Biol Chem 276:20882-20889.
16. Suzuki-Inoue,K., Y.Ozaki, M.Kainoh, Y.Shin, Y.Wu, Y.Yatomi, T.Ohmori, T.Tanaka, K.Satoh, and T.Morita. 2001. Rhodocytin induces platelet aggregation by interacting with glycoprotein Ia/IIa (GPIa/IIa, Integrin alpha 2beta 1). Involvement of GPIa/IIa-associated src and protein tyrosine phosphorylation. J Biol Chem 276:1643-1652.
17. Chung,C.H., H.C.Peng, and T.F.Huang. 2001. Aggretin, a C-type lectin protein, induces platelet aggregation via integrin alpha(2)beta(1) and GPIb in a phosphatidylinositol 3-kinase independent pathway. Biochem.Biophys.Res.Commun. 285:689-695.
18. Bergmeier,W., D.Bouvard, J.A.Eble, R.Mokhtari-Nejad, V.Schulte, H.Zirngibl. C.Brakebusch, R.Fassler, and B.Nieswandt. 2001. Rhodocytin (aggretin) activates platelets lacking alpha(2)beta(1) integrin, glycoprotein VI, and the ligand-binding domain of glycoprotein Ibalpha. J Biol Chem 276:25121-25126.
19. Honn,K.V., D.G.Tang, and J.D.Crissman. 1992. Platelets and cancer metastasis: a causal relationship? Cancer Metastasis Rev 11:325-351.
20. Nieswandt,B., M.Hafner, B.Echtenacher, and D.N.Mannel. 1999. Lysis of tumor cells by natural killer cells in mice is impeded by platelets. Cancer Res 59:1295-1300.
21. Suzuki-Inoue,K., Y.Kato, O.Inoue, M.K.Kaneko, K.Mishima, Y.Yatomi, H.Narimatsu, and Y.Ozaki. 2007. Involvement of the snake toxin receptor CLEC-2 in podoplanin-mediated platelet activation by cancer cells. J.Biol.Chem. 282:25993-26001.
22. Chaipan,C., E.J.Soilleux, P.Simpson, H.Hofmann, T.Gramberg, A.Marzi, M.Geier, E.A.Stewart, J.Eisemann, A.Steinkasserer, K.Suzuki-Inoue, C.L.Fuller, A.C.Pearce, S.P.Watson, J.A.Hoxie, F.Baribaud, and S.Pohlmann. 2006. DC-SIGN and CLEC-2 mediate human immunodeficiency virus type 1 capture by platelets, J.Virol. 80:8951-8960.
23. Kerrigan,A.M., K.M.Dennehy, D.Mourao-Sa, I.Faro-Trindade, J.A.Willment, P.R.Taylor, J.A.Eble, Reis e Sousa, and G.D.Brown. 2009. CLEC-2 is a phagocytic activation receptor expressed on murine peripheral blood neutrophils. J.Immunol, 182:4150-4157.
24. O'Callaghan,C.A. 2009. Thrombomodulation via CLEC-2 targeting. Curr.Opin.Pharmacol. 9:90-95.
25. Clemetson,J.M., J.Polgar, E.Magnenat, T.N.Wells, and K.J.Clemetson. 1999. The platelet collagen receptor glycoprotein VI is a member of the immunoglobulin superfamily closely related to FcalphaR and the natural killer receptors. J Biol Chem 274:29019-29024.
26. Gruner,S., M.Prostredna, M.Koch, Y.Miura, V.Schulte, S.M.Jung, M.Moroi, and B.Nieswandt. 2005. Relative antithrombotic effect of soluble GPVI dimer compared with anti-GPVI antibodies in mice. Blood 105:1492-1498.
27. Nieswandt,B., W.Bergmeier, V.Schulte, K.Rackebrandt, J.E.Gessner, and H.Zirngibl. 2000. Expression and function of the mouse collagen receptor glycoprotein VI is strictly dependent on its association with the FcRgamma chain. J Biol Chem 275:23898-24002.
28. Nieswandt,B., C.Brakebusch, W.Bergmeier, V.Schulte, D.Bouvard, R.Mokhtari-Nejad, T.Lindhout, J.W.Heemskerk, H.Zirngibl, and R.Fassler. 2001. Glycoprotein VI but not alpha2beta1 integrin is essential for platelet interaction with collagen. EMBO J 20:2120-2130.
29. Ruggeri,Z.M. and G.L.Mendolicchio. 2007. Adhesion mechanisms in platelet function. Circ.Res. 100:1673-1685.
30. Nieswandt,B., V.Schulte, W.Bergmeier, R.Mokhtari-Nejad, K.Rackebrandt, J.P.Cazenave, P.Ohlmann, C.Gachet, and H.Zirngibl. 2001. Long-term Antithrombotic Protection by In Vivo Depletion of Platelet Glycoprotein VI in Mice. J Exp Med 193:458-470.
31. Boylan,B., V.Rathore, C.Paddock, K.Friedman, B.Curtis, M.Stapleton, D.K.Newman, H.Chen, M.Kahn, and P.J.Newman. 2003. JAQ-2/3-iike ITP - a newly recognized clinical syndrome caused by autoantibody-mediated clearance of the GPVI/FcR gamma-chain complex from the human platelet surface. Blood 102:12A,
32. Xie,J., T.Wu, L.Guo, Y.Ruan, L.Zhou, H.Zhu, X.Yun, Y.Hong, J.Jiang, Y.Wen, and J.Gu. 2008. Molecular characterization of two novel isoforms and a soluble form of mouse CLEC-2. Biochem.Biophys.Res.Commun. 371:180-184.
33. Rabie,T., D.Varga-Szabo, M.Bender, R.Pozgaj, F.Lanza, T.Saito, S.P.Watson, and B.Nieswandt. 2007. Diverging signaling events control the pathway of GPVI down-regulation in vivo. Blood 110:529-535.
34. Schulte,V., T.Rabie, M.Prostredna, B.Aktas, S.Gruner, and B.Nieswandt. 2003. Targeting of the collagen-binding site on glycoprotein VI is not essential for in vivo depletion of the receptor. Blood 101:3948-3952.
35. Boylan,B., M.C.Berndt, M,L.Kahn, and P.J.Newman. 2006. Activation. independent, antibody-mediated removal of GPVI from circulating human platelets: development of a novel NOD/SCID mouse model to evaluate the in vivo effectiveness of anti-human platelet agents. Blood 108:908-914.
36. Varga-Szabo,D., A.Braun, C.Kleinschnitz, M.Bender, I.Pleines, M.Pham, T.Renne, G.Stoll, and B.Nieswandt. 2008. The calcium sensor STIM1 is an essential mediator of arterial thrombosis and ischemic brain infarction. J. Exp.Med.205:1583-1591.
37. Grosse,J., A.Braun, D.Varga-Szabo, N.Beyersdorf, B.Schneider, L.Zeitlmann, P.Hanke, P.Schropp, S.Muhlstedt, C.Zom, M.Huber, C.Schmittwolf, W.Jagla, P.Yu, T.Kerkau, H.Schulze, M.Nehls, and B.Nieswandt. 2007. An EF hand mutation in Stim1 causes premature platelet activation and bleeding in mice. J.Clin.lnvest 117:3540-3550.
38. Judd,B.A., P.S.Myung, A.Obergfell, E.E.Myers, A.M.Cheng, S.P.Watson, W.S.Pear, D.Allman, S.J.Shattil, and G.A.Koretzky. 2002. Differential requirement for LAT and SLP-76 in GPVI versus T cell receptor signaling. J.Exp.Med. 195:705-717.
39. Mangin,P., C.Nonne, A.Eckly, P.Ohlmann, M.Freund, B.Nieswandt, J.P.Cazenave, C.Gachet, and F.Lanza. 2003. A PLC gamma 2-independent platelet collagen aggregation requiring functional association of GPVI and integrin alpha2beta1. FEBS Lett. 542:53-59.
40. Kleinschnitz,C., M.Pozgajova, M.Pham, M.Bendszus, B.Nieswandt, and G.Stoll. 2007. Targeting platelets in acute experimental stroke: impact of glycoprotein Ib, VI, and IIb/IIIa blockade on infarct size, functional outcome, and intracranial bleeding. Circulation 115;2323-2330.
41. Rodgers.R.P. and J.Levin. 1990. A critical reappraisal of the bleeding time. Semin.Thromb.Hemost. 16:1-20.
42. Moroi,M., S.M.Jung, M.Okuma, and K.Shinmyozu. 1989. A patient with platelets deficient in glycoprotein VI that lack both collagen-induced aggregation and adhesion. J Clin Invest 84:1440-1445.
43. Bergmeier,W., V.Schulte, G.Brockhoff, U.Bier, H.Zirngibl, and B.Nieswandt. 2002. Flow cytometric detection of activated mouse integrin alphallbbeta3 with a novel monoclonal antibody. Cytometry 48:80-86.
44. Papathanasiou, A., Goudevenos, J., Tselepis, A.D. Resistance to Aspirin and Clopidogrel: Possible Mechanisms, Laboratory Investigation, and Clinical Significance. Hellenic J Cardiol 48: 352-363, 2007

## Claims

1. A pharmaceutical composition comprising an inhibitor of C-type lectin-like receptor 2 (CLEC-2) or an inhibitor of an activator of CLEC-2 and optionally a pharmaceutically acceptable carrier, excipient and/or diluent.

2. An inhibitor of C-type lectin-like receptor 2 (CLEC-2) or an inhibitor of an activator of CLEC-2 for use in treating or preventing a disorder related to venous or arterial thrombus formation or as a lead compound for developing a drug for treating or preventing a disorder related to venous or arterial thrombus formation.

3. A method of treating and/or preventing a disorder related to venous or arterial thrombus formation comprising administering a pharmaceutically effective amount of an inhibitor of C-type lectin-like receptor 2 (CLEC-2) or an inhibitor of an activator of CLEC-2 to a subject in need thereof.

4. The pharmaceutical composition of claim 1 or the inhibitor of claim 2 or the method of claim 3, wherein the inhibitor is an antibody or a fragment or derivative thereof, an aptamer, a siRNA, a shRNA, a miRNA, a ribozyme, an antisense nucleic acid molecule or a small molecule.

5. The pharmaceutical composition of claim 1 or 4 or the inhibitor of claim 2 or 4 or the method of claim 3 or 4, wherein the activator of CLEC-2 is podoplanin, rhodocytin or a derivative thereof.

6. The pharmaceutical composition of any one of claims 1 and 4 to 5 further comprising in the same or a separate container an antagonist of G-protein coupled receptors or signaling pathways.

7. The inhibitor of any one of claims 2 and 4 to 5 having admixed thereto or associated in a separate container an antagonist of G-protein coupled receptors or signaling pathways.

8. The method of any one of claims 3 to 5, further comprising the administration of an antagonist of G-protein coupled receptors or signaling pathways.

9. The pharmaceutical composition of claim 6 or the inhibitor of claim 7 or the method of claim 8, wherein the antagonist of G-protein coupled receptors or signaling pathways is aspirin or is an inhibitor of P2Y1, P2Y12 or PAR receptors.

10. The inhibitor of any one of claims 2, 4, 5, 7 and 9 or the method of any one of claims 3 to 5, 8 and 9, wherein the disorder related to venous or arterial thrombus formation is selected from the group consisting of myocardial infarction, stroke, ischemic stroke, pulmonary thromboembolism, peripheral artery disease (PAD), arterial thrombosis and venous thrombosis.

11. The inhibitor or the method of claim 10, wherein said stroke is ischemic stroke.

12. A method of identifying a compound suitable as a lead compound and/or as a medicament for the treatment and/or prevention of a disorder related to venous or arterial thrombus formation, comprising the steps of:
(a) determining the level of CLEC-2 protein or *Clec-2* transcript in thrombocytes;
(b) contacting said thrombocytes or thrombocytes of the same population with a test compound;
(c) determining the level of CLEC-2 protein or Clec-2 transcript in said thrombocytes after contacting with the test compound; and
(d) comparing the level of CLEC-2 protein or Clec-2 transcript determined in step (c) with the CLEC-2 protein or *Clec*-2 transcript level determined in step (a), wherein a decrease of CLEC-2 protein or *Clec*-2 transcript level in step (c) as compared to step (a) indicates that the test compound is a compound suitable as a lead compound and/or as a medicament for the treatment and/or prevention of a disorder related to venous or arterial thrombus formation.

13. A method of identifying a compound suitable as a lead compound and/or as a medicament for the treatment and/or prevention of a disorder related to venous or arterial thrombus formation, comprising the steps of:
(a) determining the level of CLEC-2-mediated activation of thrombocytes upon stimulation of the thrombocytes with a CLEC-2 ligand;
(b) contacting said thrombocytes or thrombocytes of the same population with a test compound;
(c) determining the level of CLEC-2-mediated activation of thrombocytes upon stimulation of the thrombocytes with a CLEC-2 ligand after contacting with the test compound; and
(d) comparing the level of CLEC-2-mediated activation of thrombocytes upon stimulation of the thrombocytes with a CLEC-2 ligand determined in step (c) with the level of CLEC-2-mediated activation of thrombocytes upon stimulation of the thrombocytes with a CLEC-2 ligand determined in step (a), wherein a decrease in the level of CLEC-2-mediated activation of thrombocytes in step (c) as compared to step (a) indicates that the test compound is a compound suitable as a lead compound and/or as a medicament for the treatment and/or prevention of a disorder related to venous or arterial thrombus formation.

14. The method of claim 13, wherein the activation of thrombocytes is determined by measuring the amount of thrombocyte aggregation, by determining the amount of fibrinogen binding and/or by measuring the amount of surface exposure of p-selectin.

15. The method of claim 13 or 14, wherein the CLEC-2 ligand is podoplanin or rhodocytin.
